(19) Europäisches Patentamt
European Patent Office
Office européen des brevets

(11) Numéro de publication: **0 300 850 B1**

(12) **FASCICULE DE BREVET EUROPEEN**

(45) Date de publication de fascicule du brevet: **26.08.92**

(51) Int. Cl.5: **C07D 207/48**, A61K 31/40, C07D 207/38, C07D 409/12, C07D 401/12

(21) Numéro de dépôt: **88401585.0**

(22) Date de dépôt: **23.06.88**

Le dossier contient des informations techniques présentées postérieurement au dépôt de la demande et ne figurant pas dans le présent fascicule.

# (54) Dérivés de 1-arylsulfonyl 1,5-dihydro 2H-pyrrol-2-one, leurs procédé et intermédiaires de préparation, leur application comme médicaments et les compositions les renfermant.

(30) Priorité: **24.06.87 IT 2102987**

(43) Date de publication de la demande: **25.01.89 Bulletin 89/04**

(45) Mention de la délivrance du brevet: **26.08.92 Bulletin 92/35**

(84) Etats contractants désignés: **AT BE CH DE ES FR GB GR IT LI LU NL SE**

(56) Documents cités:
**EP-A- 0 138 721**

**CHEMICAL ABSTRACTS, vol. 102, 1985, pages 602-603, résumé N 149188z, Columbus, Ohio, US; K. YOSHIYUKI et al.: "1,3-Dipolar cycloaddition of aryl azides with 5-alkoxy-3-pyrrolin-2-ones. Synthesis of 4-alkoxy-6-aryl-3,6-diazabicyclo[3.1.0]hexan-2-ones"**

(73) Titulaire: **ROUSSEL-UCLAF**
**35, Boulevard des Invalides**
**F-75007 Paris(FR)**

(72) Inventeur: **Toja, Emilio**
**Via Plezzo 80**
**Milano(IT)**
Inventeur: **Gorini, Carlo**
**Via Orcagna 4**
**Milano(IT)**
Inventeur: **Barzaghi, Fernando**
**Via Monteverdi 21**
**I-20052 Monza - Milan(IT)**
Inventeur: **Galliani, Giulio**
**13, Via Silva**
**1 Monza (MI)(IT)**

(74) Mandataire: **Tonnellier, Marie-José et al**
**111, route de Noisy B.P. no 9**
**F-93230 Romainville(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

**JOURNAL OF HETEROCYCLIC CHEMISTRY**, vol. 24, N 5, septembre-octobre 1987, pages 1269-1274; F. FARINA et al.: "Pseudoesters and derivatives. XXV[1]. 1,3-Dipolar cycloaddition of diazomethane to 5-methoxy-3-pyrrolin-2-ones"

**CHEMICAL ABSTRACTS**, vol. 83, 1975, page 801, résumé N 9666t, Columbus, Ohio, US; W. FLITSCH et al.: "Stable solution of salts of 1-aza-2-cyclopentadienone in thionyl chloride-antimony pentachloride"

**DRUGS OF THE FUTURE**, vol. 10, N 12, 1985, pages 972-974

## Description

L'invention concerne de nouveaux dérivés de 1-arylsulfonyl 1,5-dihydro 2H-pyrrol 2-one, leurs procédé et intermédiaires de préparation, leur application comme médicaments et les compositions les renfermant.

On connaissait des 4-hydroxy ou 4-acyloxy 1-benzènesulfonyl 2-oxopyrrolidines du brevet européen EP.A. 138721 utilisables dans le traitement des troubles de la mémoire ou du surmenage intellectuel ; on vient de découvrir des composés de structure chimique voisine présentant d'intéressantes propriétés.

L'invention a pour objet les composés répondant à la formule générale (I) :

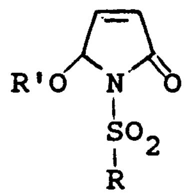

(I)

dans laquelle R' représente un atome d'hydrogène, un radical alcoyle, linéaire, ramifié ou cyclique renfermant jusqu'à 8 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou naphtyle ou un radical thiényle, furyle, pyrannyle, pyridyle, benzofurannyle, isobenzofurannyle, chromanyle, isochromanyle, chroményle, xanthényle, phénoxathiényle, oxazolyle, isoxazolyle, furazanyle, phénoxazinyle, thiéno[2,3-b]furannyle, 2H-furo[3,2-b]pyrannyle, benzoxazolyle ou morpholinyle, le radical R étant éventuellement substitué par un ou plusieurs substituants choisis dans le groupe constitué par le radical hydroxy, le radical acétoxy, le radical méthoxy ou le radical benzyloxy, les radicaux alcoyles linéaires, ramifiés ou cycliques, saturés ou insaturés, comportant jusqu'à 18 atomes de carbone, les atomes d'halogène, les groupements $CF_3$, $SCF_3$, $OCF_3$, $NO_2$, $NH_2$ ou C≡N, le radical phényle, les groupements acyles ou alcoxycarbonyles renfermant de 2 à 8 atomes de carbone et les groupements alcoylsulfonyles renfermant de 1 à 6 atomes de carbone.

Par radical alcoyle, on entend de préférence un radical renfermant de 1 à 6 atomes de carbone, par exemple, le radical méthyle, éthyle, n-propyle, isopropyle, n-butyle, isobutyle, terbutyle, n-pentyle, n-hexyle, cyclopropyle, cyclobutyle, cyclopentyle ou cyclohexyle.

Par radical alcényle, on entend de préférence un radical éthényle, propényle, butényle.

Par radical acyle, on entend de préférence un radical acétyle, propionyle ou butyryle.

Lorsque le radical R est substitué par un ou plusieurs radicaux alcoyles linéaires, saturés ou insaturés, il s'agit de préférence d'un ou plusieurs radicaux méthyle, éthyle, propyle ou isopropyle, éthényle ou éthynyle.

Lorsque le radical R est substitué par un ou plusieurs atomes d'halogène, l'halogène est de préférence le fluor, le chlore ou le brome.

L'invention a notamment pour objet les composés de formule (I) dans lesquels R représente un radical phényle éventuellement substitué par l'un des substituants indiqués précédemment.

L'invention a aussi notamment pour objet les composés de formule (I) dans lesquels R' représente un radical alcoyle linéaire, ramifié ou cyclique, renfermant jusqu'à 8 atomes de carbone et plus particulièrement un radical n-butyle ou n-pentyle.

L'invention a tout spécialement pour objet la 1-benzènesulfonyl 1,5-dihydro 5n-butoxy 2H-pyrrol 2-one, la 1-benzènesulfonyl 1,5-dihydro 5n-pentyloxy 2H-pyrrol 2-one et la 1-(3-trifluorométhyl benzènesulfonyl) 1,5-dihydro 5n-butoxy 2H-pyrrol 2-one.

Les composés de l'invention présentent d'intéressantes propriétés pharmacologiques : ils retardent l'extinction de la réponse d'évitement conditionné, ils retardent la disparition de la réponse apprise. Ils favorisent l'attention, la vigilance et la mémorisation.

L'invention a donc pour objet les produits de formule (I) en tant que médicaments, utiles notamment dans le traitement des asthénies intellectuelles ou nerveuses, des défaillances de la mémoire, de la sénescence, du surmenage intellectuel.

L'invention a plus particulièrement pour objet en tant que médicament, les produits préférés mentionnés précédemment et notamment les produits des exemples 4, 6 et 12.

La posologie usuelle est variable selon l'affection en cause, le sujet traité et la voie d'administration, elle peut être comprise entre 50 mg et 3000 mg/jour, par exemple entre 150 et 1500 mg/jour en une ou plusieurs prises pour le produit de l'exemple 4 administré par voie orale.

La présente invention a également pour objet les compositions pharmaceutiques renfermant comme principe actif au moins un produit de formule (I).

Les compositions pharmaceutiques de l'invention peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule (I), caractérisé en ce que l'on soumet la 1,5-dihydro 5-hydroxy 2H-pyrrol 2-one de formule :

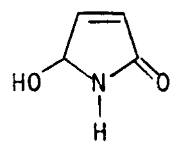

à l'action d'un alcool de formule (II):

R'-OH (II)

R' étant comme défini comme précédemment, pour obtenir le composé de formule (III):

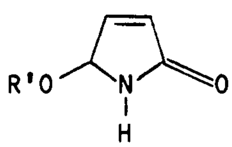

(III)

que l'on soumet à l'action d'un composé de formule (IV):

$RSO_2$-Hal (IV)

dans laquelle Hal représente un atome de chlore ou de brome et R conserve sa signification précédente, pour obtenir le composé de formule (I) correspondant.

Dans un mode de réalisation préférée:

- la condensation de la 1,5-dihydro 5-hydroxy 2H-pyrrol 2-one et de l'alcool de formule (11) a lieu en présence de résine Amberlite®
- la condensation entre le produit de formule (III) et le produit de formule (IV) est effectuée:
  a) en présence d'une base forte comme le butyllithium, un hydrure alcalin, comme l'hydrure de sodium ou le bis-(triméthylsilyl) amidure de sodium;
  b) au sein d'un solvant choisi dans le groupe constitué par le tétrahydrofuranne, le benzène, le diméthylformamide, le diméthylsulfoxyde ou l'éther diéthylique du diéthylène glycol.

La 1,5-dihydro 5-hydroxy 2H-pyrrol 2-one est un produit décrit dans Synthesis 1973, p. 167.

Les exemples suivants illustrent l'invention sans toutefois la limiter.

**Exemple 1 : 1-benzènesulfonyl 1,5-dihydro 5-éthoxy 2H-pyrrol 2-one.**

**Stade A** : 1,5-dihydro 5-éthoxy 2H-pyrrol 2-one.

On porte 30 minutes au reflux un mélange de 2,3 g de 1,5-dihydro 5-hydroxy 2H-pyrrol 2-one dans 140 cm3 d'éthanol et 100 g de résine Amberlite® IR-120 (H). On laisse refroidir, filtre la résine et distille l'éthanol sous pression réduite. On obtient 2,76 g de produit attendu. F = 50-52¤C.

**Stade B** : 1-benzènesulfonyl 1,5-dihydro 5-éthoxy 2H-pyrrol 2-one.

A une solution de 1,5 g de 1,5-dihydro 5-éthoxy 2H-pyrrol 2-one dans 40 cm3 de tétrahydrofuranne refroidie à -35¤C, on ajoute 7,37 cm3 d'une solution 1,6M de n-butyllithium dans le n-hexane en opérant à -35¤/-30¤C. On agite pendant 20 minutes puis refroidit à -40¤C et en maintenant la température entre -38¤C et -40¤C, on ajoute une solution de 2,08 g de chlorure de benzènesulfonyle dans 10 cm3 de tétrahydrofuranne. On laisse revenir à température ambiante, évapore le solvant sous pression réduite et chromatographie le résidu sur silice (éluant : acétate d'éthyle-n-hexane 1-1). On obtient 1,15 g de produit attendu. F = 117-119¤C cristallisé de l'éther éthylique.

**Exemple 2 : 1-benzènesulfonyl 1,5-dihydro 5-n-propyloxy 2H-pyrrol 2-one.**

**Stade A** : 1,5-dihydro 5-n-propyloxy 2H-pyrrol 2-one.

On porte à 60¤C pendant 2 heures 30 minutes un mélange de 3 g de 1,5-dihydro 5-hydroxy 2H-pyrrol 2-one dans 60 cm3 de n-propanol et 1,5 g d'Amberlite® IR 120 H. On laisse refroidir, filtre, évapore à sec et chromatographie sur silice (éluant : acétate d'éthyle). On obtient 3,3 g de produit attendu que l'on utilise tel quel pour le stade suivant.

**Stade B** : 1-benzènesulfonyl 1,5-dihydro 5-n-propyloxy 2H-pyrrol 2-one.

A une solution de 3 g de 1,5-dihydro 5-n-propyloxy 2H-pyrrol 2-one dans 80 cm3 de tétrahydrofuranne, refroidie à -45¤C, on ajoute 13,25 cm3 d'une solution 1,61M de n-butyllithium dans le n-hexane en maintenant la température entre -35¤C et -45¤C. Après 30 minutes, on ajoute une solution de 3,74 g de chlorure de benzènesulfonyle dans 12 cm3 de tétrahydrofuranne à une température comprise entre -45¤C et -35¤C, laisse revenir à température ambiante pendant 2 heures. On évapore à sec, chromatographie sur silice (éluant : acétate d'éthyle-n-hexane 1-1). On recueille 2,2 g de produit attendu. F = 56-58¤C cristallisé d'un mélange éther éthylique-n-hexane 1-2). Par cristallisation dans l'isopropanol, on obtient 1,4 g de produit attendu. F = 66-67¤C.

**Exemple 3 : 1-benzènesulfonyl 1,5-dihydro 5-isopropyloxy 2H-pyrrol 2-one.**

**Stade A** : 1,5-dihydro 5-isopropyloxy 2H-pyrrol 2-one.

On porte à 45-50¤C pendant 3 heures un mélange de 3 g de 1,5-dihydro 5-hydroxy 2H-pyrrol 2-one, 1,5 g d'Amberlite® IR 120 H et 60 cm3 d'isopropanol. Après refroidissement, on filtre et évapore à sec. On obtient 1,8 g de produit attendu. F = 79-81¤C cristallisé de l'isopropanol.

**Stade B** : 1-benzènesulfonyl 1,5-dihydro 5-isopropyloxy 2H-pyrrol 2-one.

A une solution de 3 g de produit obtenu au stade A dans 80 cm3 de tétrahydrofuranne, on ajoute à -45¤C, 13,2 cm3 d'une solution 1,6M de n-butyl lithium dans l'hexane en maintenant la température entre -45¤C et 40¤C. On agite pendant 30 minutes et ajoute une solution de 3,74 g de chlorure de benzènesulfonyle dans 12 cm3 de tétrahydrofuranne en maintenant la température à -45¤C/-40¤C. On laisse revenir à température ambiante pendant 2 heures, évapore à sec, reprend avec 50 cm3 d'eau, filtre et obtient 1,65 g de produit attendu. F = 148-149¤C cristallisé de l'isopropanol.

**Exemple 4 : 1-benzènesulfonyl 5-n-butoxy 1,5-dihydro 2H-pyrrol 2-one.**

**Stade A :** 5-n-butoxy 1,5-dihydro 2H-pyrrol 2-one.

On chauffe à 70¤C pendant 2 heures un mélange de 2,5 g de 1,5-dihydro 5-hydroxy 2H-pyrrol 2-one dans 75 cm3 de 1-butanol et 1,3 g d'Amberlite® IR 120 H. On laisse revenir à température ambiante, filtre le résidu et évapore le solvant sous pression réduite. On obtient 3,9 g de produit attendu.

**Stade B** : 1-benzènesulfonyl 5-n-butoxy 1,5dihydro 2H-pyrrol 2-one.

A une solution de 3,9 g de produit obtenu au stade A dans 80 cm3 de tétrahydrofuranne, refroidie à -35¤C, on ajoute 15,7 cm3 d'une solution 1,6M de n-butyllithium dans le n-hexane en maintenant la température entre - 35¤C et -30¤C. On agite pendant 20 minutes puis on ajoute à -40¤C/-38¤C une

solution de 4,44 g de chlorure de benzènesulfonyle dans 20 cm3 de tétrahydrofuranne. On laisse revenir à température ambiante, évapore le solvant sous pression réduite et chromatographie le résidu sur silice (éluant : n-hexane-acétate d'éthyle 2-1). On obtient 1,9 g de produit attendu. F = 66-67¤C cristallisé de l'éther isopropylique.

**Exemple 5 : 1-benzènesulfonyl 1,5-dihydro 5-isobutyloxy 2H-pyrrol 2-one.**

**Stade A :** 1,5-dihydro 5-isobutyloxy 2H-pyrrol 2-one.

On chauffe à 50¤C pendant 3 heures un mélange de 3 g de 1,5-dihydro 5-hydroxy 2H-pyrrol 2-one, 1,5 g d'Amberlite® IR 120 H dans 60 cm3 d'isobutanol. On laisse refroidir, filtre et évapore le solvant. Après chromatographie du résidu sur silice (éluant : acétate d'éthyle), on obtient 4 g de produit attendu.

**Stade B :** 1-benzènesulfonyl 1,5-dihydro 5-isobutyloxy 2H-pyrrol 2-one.

A une solution refroidie à -45¤C de 3,3 g de 1,5-dihydro 5-isobutyloxy 2H-pyrrol 2-one obtenue au stade A dans 80 cm3 de tétrahydrofuranne, on ajoute 13,2 cm3 d'une solution 1,6M de n-butyllithium dans l'hexane. On agite pendant 30 minutes à -45¤C/-40¤C puis ajoute une solution de 3,74 g de chlorure de benzènesulfonyle dans 12 cm3 de tétrahydrofuranne. On laisse revenir à température ambiante Pendant 2 heures. On évapore à sec et chromatographie le résidu sur silice (éluant : acétate d'éthyle-n-hexane 1-1). On obtient 1,7 g de produit attendu. F = 82-83¤C cristallisé de l'isopropanol.

**Exemple 6 : 1-benzènesulfonyl 1,5-dihydro 5-n-pentyloxy 2H-pyrrol 2-one.**

**Stade A :** 1,5-dihydro 5-n-pentyloxy 2H-pyrrol 2-one.

On chauffe à 50¤C pendant 3 heures un mélange de 3 g de 1,5-dihydro 5-hydroxy 2H-pyrrol 2-one dans 60 cm3 d'alcool n-pentylique et 1,5 g d'Amberlite® IR 120 H. On laisse revenir à température ambiante, filtre et distille le solvant sous pression réduite. Le résidu est distillé à 200¤C sous 0,08 mbar. On obtient 3,8 g de produit attendu utilisé tel quel pour le stade suivant.

**Stade B :** 1-benzènesulfonyl 1,5-dihydro 5-n-pentyloxy 2H-pyrol 2-one.

A une solution à -45¤C de 3,5 g du produit obtenu au stade A dans 80 cm3 de tétrahydrofuranne, on ajoute à -40¤C/-45¤C 13,8 c d'une solution 1,5M de n-butyllithium dans l'hexane. Après 30 minutes, on ajoute à -45¤C/-40¤C une solution de 3,66 g de chlorure de benzènesulfonyle dans 12 cm3 de tétrahydrofuranne. On laisse revenir à température ambiante 2 heures. On évapore à sec puis chromatographie sur silice (éluant : acétate d'éthyle-n-hexane1-1). On obtient 2,5 g de produit attendu.

**Exemple 7 : 1-benzènesulfonyl 1,5-dihydro 5-n-hexyloxy 2H-pyrrol 2-one.**

**Stade A :** 1,5-dihydro 5-n-hexyloxy 2H-pyrrol 2-one.

On chauffe à 85¤C pendant 2 heures un mélange de 2,5 g de 1,5-dihydro 5-hydroxy 2H-pyrrol 2-one dans 75 cm3 de 1-hexanol et 1,3 g d'Amberlite® IR 120 H. On laisse refroidir à température ambiante, filtre la résine et évapore le solvant sous pression réduite, distille le résidu et obtient 3,4 g de produit attendu. Eb 120-125¤C sous 0,4 mbar.

**Stade B :** 1-benzènesulfonyl 1,5-dihydro 5-n-hexyloxy 2H-pyrrol 2-one.

A une solution de 10,1 g de 1,5-dihydro 5-n-hexyloxy 2H-pyrrol 2-one obtenue au stade A dans 170 cm3 de tétrahydrofuranne refroidie à -30¤C, on ajoute 39,37 cm3 d'une solution 1,4M de n-butyllithium dans le n-hexane. On maintient la température à -30¤C. On agite 15 minutes, refroidit à -35¤C et ajoute une solution de 9,73 g de chlorure de benzènesulfonyle dans 70 cm3 de tétrahydrofuranne en maintenant la température entre -35¤C et -53¤C. On laisse revenir à température ambiante, évapore le solvant sous pression réduite et chromatographie le résidu sur silice (éluant : acétate d'éthyle-n-hexane 1-2). On obtient 5,68 g de produit attendu.

**Exemple 8 : 1-benzènesulfonyl 1,5-dihydro 5-cyclopentyloxy 2H-pyrrol 2-one.**

**Stade A :** 1,5-dihydro 5-cyclopentyloxy 2H-pyrrol 2-one.

On maintient 8 heures à 60¤C un mélange de 4,5 g de 1,5-dihydro 5-hydroxy 2H-pyrrol 2-one, 100 cm3 de cyclopentanol et 2,25 g d'Amberlite® IR 120 H. On laisse refroidir, filtre et évapore à sec, chromatographie le résidu sur silice (éluant : acétate d'éthyle-n-hexane 1-1). On obtient 3 g de produit attendu. F = 77-78¤C cristallisé du cyclohexane. On le recristallise dans le mélange éther isopropylique-n-hexane 1-1. F = 81-82¤C.

**Stade B :** 1-benzènesulfonyl 1,5-dihydro 5-cyclopentyloxy 2H-pyrrol 2-one.

A une solution refroidie à -40¤C de 4 g de produit obtenu au stade A dans 160 cm3 de tétrahydrofuranne, on ajoute à -40¤C 15 cm3 d'une solution 1,6M de butyllithium dans l'hexane. Après 30 minutes à -40¤C, on ajoute une solution de 4,2 g de chlorure de benzènesulfonyle dans 30 cm3 de tétrahydrofuranne. On laisse revenir à température ambiante, évapore à sec, reprend le résidu avec un mélange eau-alcool isopropylique (1-3). On obtient 2,06 g de produit attendu. F = 123-124¤C.

**Exemple 9 : 1-benzènesulfonyl 1,5-dihydro 5-cyclohexyloxy 2H-pyrrol 2-one.**

**Stade A :** 1,5-dihydro 5-cyclohexyloxy 2H-pyrrol 2-one.

On chauffe à 90¤C pendant 3 heures un mélange de 5 g de 1,5-dihydro 5-hydroxy 2H-pyrrol 2-one dans 150 cm3 de cyclohexane avec 2,5 g d'Amberlite® IR 120 H. On filtre, évapore le solvant sous pression réduite et chromatographie le résidu sur silice (éluant : acétate d'éthyle-n-hexane 1-1). On obtient 4,98 g de produit attendu. F = 65-66¤C.

**Stade B :** 1-benzènesulfonyl 1,5-dihydro 5-cyclohexyloxy 2H-pyrrol 2-one.

A une solution de 2 g de 5-cyclohexyloxy 1,5-dihydro 2H-pyrrol 2-one obtenue au stade A dans 35 cm3 de tétrahydrofuranne refroidie à -40¤C, on ajoute 8,83 cm3 d'une solution 1,25M de n-butyllithium dans l'hexane en maintenant la température à -40¤C/-35¤C. On agite 20 minutes puis refroidit à -45¤C et ajoute une solution de 1,95 g de chlorure de benzènesulfonyle dans 15 cm3 de tétrahydrofuranne. On laisse revenir à température ambiante, évapore le solvant sous pression réduite et chromatographie le résidu sur silice (éluant : n-hexane-acétate d'éthyle 3-1). On obtient 1 g de produit attendu. F = 104-106¤C cristallisé de l'éther isopropylique.

**Exemple 10 : 1-benzènesulfonyl 1,5-dihydro 5-benzyloxy 2H-pyrrol 2-one.**

**Stade A :** 1,5-dihydro 5-benzyloxy 2H-pyrrol 2-one.

On chauffe 3 heures à 60¤C un mélange de 8 g de 1,5-dihydro 5-hydroxy 2H-pyrrol 2-one dans 130 cm3 d'alcool benzylique et 4 g d'Amberlite® IR 120 H. On distille l'alcool benzylique à 65¤C sous 0,3 mbar, puis l'entraîne sous forme d'azeotrope à l'eau puis au benzène. On obtient 13,6 g de produit attendu instable à la distillation.

**Stade B :** 1-benzènesulfonyl 1,5-dihydro 5-benzyloxy 2H-pyrrol 2-one.

A une solution de 1,83 g de bis-triméthylsilylamidure de sodium dans 91 cm3 d'éther éthylique, on ajoute une solution de 1,7 g de produit obtenu au stade A dans 4 cm3 d'éther éthylique. On agite pendant 30 minutes puis refroidit à 0¤C et ajoute une solution de 1,51 g de chlorure de benzènesulfonyle dans 5 cm3 d'éther éthylique. On agite 2 heures à température ambiante. On coule dans 50 cm3 de chloroforme, filtre, évapore et chromatographie le résidu sur silice (éluant : acétate d'éthyle-n-hexane 1-1). On obtient 0,7 g de produit attendu. F = 56-58¤C.

**Exemple 11 : 1-(4-méthoxy benzènesulfonyl 5-n-butyloxy 1,5-dihydro 2H-pyrrol 2-one.**

A une solution de 3 g de 5-n-butyloxy 1,5-dihydro 2H-pyrrol 2-one, obtenu comme au stade A de

l'exemple 4 dans 90 cm3 de tétrahydrofuranne, on ajoute à -70¤C/-65¤C 12,06 cm3 d'une solution 1,6M de butyllithium dans le n-hexane. On agite pendant 15 minutes à -70¤C puis ajoute une solution de 4 g de chlorure de 4-méthoxy benzènesulfonyle dans 12 cm3 de tétrahydrofuranne à une température comprise entre -70¤C et -65¤C. On laisse revenir à température ambiante pendant 2 heures. On évapore à sec et chromatographie le résidu (éluant : acétate d'éthyle-n-hexane 1-1). On obtient 1,6 g de produit attendu. F = 65-66¤C cristallisé de l'éther isopropylique.

| Analyse : $C_{15}H_{19}NO_5S$ | | | |
|---|---|---|---|
| Calculé : | C% 55,37 | H% 5,89 | N% 4,3 |
| Trouvé : | 55,48 | 5,82 | 4,39 |

**Exemple 12 : 1-(3-trifluorométhyl benzènesulfonyl 5-n-butyloxy 1,5-dihydro 2H-pyrrol 2-one.**

On opère comme à l'exemple 11 en utilisant 3 g du produit obtenu comme au stade A de l'exemple 4 et en opérant avec 4,72 g de chlorure de 3-trifluorométhyl benzènesulfonyle au lieu de chlorure de 4-méthoxy benzènesulfonyle. On obtient après distillation à 200¤C sous 0,05 mbar 1,6 g de produit attendu. F = 42-43¤C.

| Analyse : $C_{15}H_{16}F_3NO_4S$ | | | |
|---|---|---|---|
| Calculé : | C% 49,58 | H% 4,44 | N% 3,85 |
| Trouvé : | 49,71 | 4,43 | 3,82 |

**Exemple 13 : 1-(4-biphénylylsulfonyl) 5-n-butyloxy 1,5-dihydro 2H-pyrrol 2-one.**

On opère comme à l'exemple 11 en utilisant 3 g de produit obtenu comme au stade A de l'exemple 4 et 4,88 g de chlorure de 4-biphénylylsulfonyle au lieu de 4-méthoxy benzènesulfonyle. On obtient 1,7 g de produit attendu. F = 112-114¤C cristallisé de l'alcool isopropylique.

| Analyse : $C_{20}H_{21}NO_4S$ | | | |
|---|---|---|---|
| Calculé : | C% 64,67 | H% 5,70 | N% 3,77 |
| Trouvé : | 64,44 | 5,62 | 3,68 |

**Exemple 14 : 1-(4-nitrobenzènesulfonyle) 5-n-butyloxy 1,5-dihydro 2H-pyrrol 2-one.**

On opère comme à l'exemple 11 en utilisant 3 g de produit obtenu comme au stade A de l'exemple 4 et 4,5 g de chlorure de 4-nitro benzènesulfonyle au lieu de chlorure de 4-méthoxy benzènesulfonyle. On obtient 2,7 g de produit attendu. F = 88-89¤C cristallisé de l'alcool isopropylique.

| Analyse : $C_{14}H_{16}N_2OS$ | | | |
|---|---|---|---|
| Calculé : | C% 49,40 | H% 4,74 | N% 8,23 |
| Trouvé : | 49,37 | 4,85 | 8,32 |

**Exemple 15 : 1-(2-thiophènesulfonyl) 5-n-butyloxy 1,5-dihydro 2H-pyrrol 2-one.**

On opère comme à l'exemple 11 en utilisant 3 g de produit obtenu comme au stade A de l'exemple 4 et 3,5 g de chlorure de 2-thiophènesulfonyle au lieu du chlorure de 4-méthoxy benzènesulfonyle. On obtient 0,3 g de produit attendu. F = 56-57¤C cristallisé de l'alcool isopropylique.

| Analyse : $C_{12}H_{15}NO_4S_2$ | | | |
|---|---|---|---|
| Calculé :<br>Trouvé : | C% 47,82<br>47,63 | H% 5,02<br>5,18 | N% 4,65<br>4,78 |

**Exemple 16 : 1-(2-pyridinesulfonyl) 5-n-butyloxy 1,5-dihydro 2H-pyrrol 2-one.**

A une solution de 6 g de 5-n-butyloxy 1,5-dihydro 2H-pyrrol 2-one obenu comme au stade A de l'exemple 4 et 180 cm3 de tétrahydrofuranne, on ajoute à -70¤C/65¤C 24,2 cm3 d'une solution 1,6M de n-butyllithium dans l'hexane. Après 15 minutes, on ajoute 100 cm3 d'une solution éthérée de chlorure de 2-pyridylsulfonyle à -70¤C/-65¤C. On laisse revenir à température ambiante pendant 2 heures et évapore à sec. On reprend le résidu avec de l'eau, décante le solvant et traite avec de l'alcool isopropylique. On obtient 2,9 g de produit attendu. F = 74-75¤C. Après recristallisation dans l'alcool isopropylique, on obtient 1,8 g de produit attendu. F = 76-77¤C.

| Analyse : $C_{13}H_{16}N_2O_4S$ | | | |
|---|---|---|---|
| Calculé :<br>Trouvé : | C% 52,68<br>52,28 | H% 5,44<br>5,31 | N% 9,45<br>9,33 |

**Exemples de compositions pharmaceutiques.**

a) On a préparé des comprimés répondant à la formule suivante :

| | |
|---|---|
| - Produit de l'exemple 4<br>- Excipient q.s. pour un comprimé terminé à<br>(Détail de l'excipient : lactose, amidon de blé, amidon traité, amidon de riz, stéarate de magnésium, talc). | 100 mg<br>300 mg |

b) On a préparé des gélules répondant à la formule suivante :

| | |
|---|---|
| - Produit de l'exemple 6<br>- Excipient q.s. pour une gélule terminée à<br>(Détail de l'excipient : talc, stéarate de magnésium, Aérosil®). | 200 mg<br>300 mg |

**ETUDE PHARMACOLOGIOUE**

**Toxicité aiguë et comportement.**

Nous avons utilisé des souris mâles ($CD_1$ Charles Rivers) d'un poids de 22-23 g à jeun depuis 16 heures. Les produits leur furent administrés normalement par voie orale aux doses de 1000 - 500 - 250 mg/kg.

L'effet des produits sur le comportement des animaux fut évalué suivant la méthode décrite par Irvin (Psychopharmacologia (1968), 13, 222-257) durant les 8 premières heures et à la 24ème heure.

La mortalité fut relevée pendant les 7 jours suivant le traitement.

La $DL_{50}$ a ainsi été trouvée supérieure à 1000 mg/kg sur les produits des exemples 1, 3 à 9 et 11 à 14.

**Apprentissage et mémorisation.**

Nous avons utilisé des souris mâles ($CD_1$ Charles Rivers) d'un poids de 25-30 g. Les animaux sont placés dans la partie lumineuse d'un box à deux compartiments communicants par une ouverture (F. Barzaghi et G. Galliani, Brit. J. Pharmacol. 86, 661 P, 1985.

A l'instant où la souris passe du compartiment lumineux au compartiment obscur, l'ouverture se ferme

et elle est immédiatement punie par une décharge éléctrique aux pattes. L'animal soumis à cette procédure apprend à mémoriser la punition. En fait, si on le remet dans le compartiment lumineux, il évitera ainsi de franchir l'ouverture et de rentrer dans le compartiment obscur.

Pour induire une amnésie rétrograde, les animaux sont soumis immédiatement après l'apprentissage à un électrochoc. Après l'électrochoc, les produits sont administrés par voie orale à différentes doses.

Nous avons utilisé de 10 à 50 animaux par dose.

L'effet antiamnésique des produits est évalué 3 heures après le traitement, en utilisant le même protocole que celui utilisé pour l'acquisition.

Le temps mis par l'animal pour retourner dans la chambre obscure (temps limite 180 secondes) est utilisé comme paramètre d'évaluation.

Dans les mêmes conditions expérimentales, les animaux témoins entrent avec un laps de temps de 40-50 secondes.

Les produits actifs sont ceux qui provoquent une augmentation significative du temps de latence avec une courbe dose-réponse en forme de cloche.

Les résultats sont exprimés en pourcentages d'augmentation du temps de latence par rapport aux témoins correspondants.

Les résultats sont les suivants :

**Pourcentage d'augmentation du temps de latence par rapport aux témoins**

| Produit de l'exemple | Dose mg/kg os | | |
|---|---|---|---|
| | 200 | 100 | 50 |
| 4 | 79* | 96* | 54* |
| 6 | 76* | 92* | 73* |
| 12 | 92* | 75* | 20* |
| PIRACETAM | 20 | 48* | 10 |

* Valeurs statistiquement différentes par rapport aux témoins.

**Conclusion :**

Les produits des exemples 4, 6 et 12 ont montré une activité anti-amnésique à des doses comprises entre 50 et 200 mg/kg.

**Revendications**

**Revendications pour les Etats contractants suivants : AT, BE, CH, DE, FR, GB, IT, LI, LU, NL, SE**

**1.** Les composés répondant à la formule générale (I) :

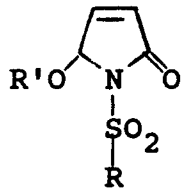

(I)

dans laquelle R' représente un atome d'hydrogène, un radical alcoyle, linéaire, ramifié ou cyclique renfermant jusqu'à 8 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou naphtyle ou un radical thiényle, furyle, pyrannyle, pyridyle, benzofurannyle, isobenzofurannyle, chromanyle, isochromanyle, chroményle, xanthényle, phénoxathiényle, oxazolyle, isoxazolyle, furazanyle, phénoxazinyle, thiéno[2,3-b]furannyle, 2H-furo[3,2-b]pyrannyle, benzoxazolyle ou morpholinyle, le radical R étant éventuellement substitué par un ou plusieurs substituants choisis dans le groupe constitué par le radical hydroxy, le radical acétoxy, le radical méthoxy ou le radical benzyloxy, les radicaux alcoyles linéaires, ramifiés ou cycliques, saturés ou insaturés, comportant jusqu'à 18 atomes de carbone, les atomes d'halogène, les groupements $CF_3$, $SCF_3$, $OCF_{31}$ $NO_2$, $NH_2$ ou C≡N, le radical phényle, les groupements acyles ou alcoxycarbonyles renfermant de 2 à 8 atomes de carbone et les groupements alcoylsulfonyles renfermant de 1 à 6 atomes de carbone.

**2.** Les composés de formule (I) tels que définis à la revendication 1, dans lesquels R représente un radical phényle éventuellement substitué par un ou plusieurs substituants définis à la revendication 1.

**3.** Les composés de formule (I) tels que définis à la revendication 1, dans lesquels R' représente un radical alcoyle linéaire, ramifié ou cyclique, renfermant jusqu'à 8 atomes de carbone.

**4.** Les composés de formule (I) tels que définis à la revendication 3, dans lesquels R' représente un radical n-butyle ou n-pentyle.

**5.** Les composés de formule (I) tels que définis à la revendication 4, dont les noms suivent :
- la 1-(benzènesulfonyl) 1,5-dihydro 5-n-butoxy 2H-pyrrol 2-one,
- la 1-(benzènesulfonyl) 1,5-dihydro 5-n-pentyloxy 2H-pyrrol 2-one,
- la 1-(3-trifluorométhyl benzènesulfonyl) 1,5-dihydro 5-n-butoxy 2H-pyrrol 2-one.

**6.** A titre de médicaments, les composés de formule (I) tels que définis à l'une quelconque des revendications 1 à 5.

**7.** A titre de médicaments, les composés définis à la revendication 5.

**8.** Les compositions pharmaceutiques renfermant comme principe actif au moins un médicament défini à la revendication 6 ou 7.

**9.** Procédé de préparation des composés de formule (I) tels que définis à l'une quelconque des revendications 1 à 5, caractérisé la 1,5-dihydro 5-hydroxy 2H-pyrrol 2-one de formule :

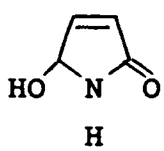

à l'action d'un alcool de formule (II) :

R'-OH (II)

R' étant défini comme précédemment, pour obtenir le composé de formule (III) :

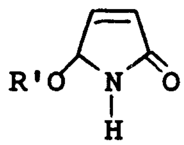

(II)

que l'on soumet à l'action d'un composé de formule (IV) :

$RSO_2$-Hal (IV)

dans laquelle Hal représente un atome de chlore ou de brome et R conserve sa signification précédente, pour obtenir le composé de formule (I) correspondant.

**Revendications pour les l'Etat contractant suivant : ES**

1. Procédé de préparation des composés répondant à la formule générale (I) :

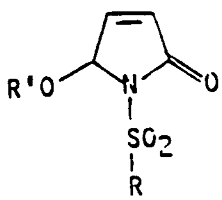

(I)

dans laquelle R' représente un atome d'hydrogène, un radical alcoyle linéaire, ramifié ou cyclique renfermant jusqu'à 8 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou naphtyle ou un radical thiényle, furyle, pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno [2,3-b] furanyle, 2H-furo [3,2-b]-pyranyle, benzoxazolyle ou morpholinyle, le radical R étant éventuellement substitué par un ou plusieurs substituants choisis dans le groupe constitué par le radical hydroxy, le radical acétoxy, le radical méthoxy ou le radical benzyloxy, les radicaux alcoyles linéaires, ramifiés ou cycliques, saturés ou insaturés, comportant jusqu'à 18 atomes de carbone, les atomes d'halogène, les groupements $CF_3$, $SCF_3$, $OCF_3$, $NO_2$, $NH_2$ ou C≡N, le radical phényle, les groupements acyles ou alcoxycarbonyles renfermant de 2 à 8 atomes de carbone et les groupements alcoylsulfonyles renfermant de 1 à 6 atomes de carbone caractérisé en ce que l'on soumet la 1,5-dihydro 5-hydroxy 2H-pyrrol 2-one de formule :

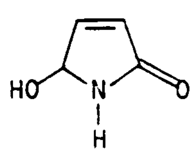

à l'action d'un alcool de formule (II) :

R'-OH (II)

R' étant défini comme précédemment, pour obtenir le composé de formule (III) :

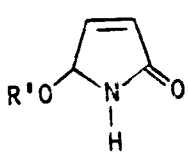

(III)

que l'on soumet à l'action d'un composé de formule (IV) :

$RSO_2$-Hal (IV)

dans laquelle Hal représente un atome de chlore ou de brome et R conserve sa signification précédente, pour obtenir le composé de formule (I) correspondant.

**2.** Procédé selon la revendication 1, caractérisé en ce que l'on utilise au départ un composé de formule (IV) dans laquelle R représente un radical phényle éventuellement substitué.

**3.** Procédé selon la revendication 1 ou 2, caractérisé en ce que l'on utilise au départ un composé de formule (II) dans laquelle R' représente un radical alcoyle linéaire, ramifié ou cyclique, renfermant jusqu'à 8 atomes de carbone.

**4.** Procédé selon la revendication 3, caractérisé en ce que l'on utilise au départ un composé de formule (III) dans laquelle R' représente un radical n-butyle ou n-pentyle.

**5.** Procédé selon la revendication 1, pour la préparation des composés dont les noms suivent :
- la 1-benzènesulfonyl 1,5-dihydro 5-n-butoxy 2H-pyrrol 2-one,
- la 1-benzènesulfonyl 1,5-dihydro 5-n-pentyloxy 2H-pyrrol 2-one,
- la 1-(3-trifluorométhyl benzènesulfonyl) 1,5-dihydro 5-n-butoxy 2H-pyrrol 2-one,

caractérisé en ce que l'on utilise au départ un composé de formule (IV) dans laquelle R représente respectivement un radical phényle ou 3-trifluorométhyl phényle et un composé de formule (II) dans laquelle R' représente respectivement un radical n-butyle ou n-pentyle.

**Revendications pour les l'Etat contractant suivant : GR**

**1.** Procédé de préparation des composés répondant à la formule générale (I) :

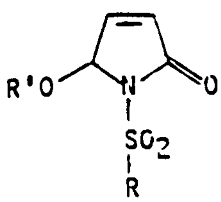

(I)

dans laquelle R' représente un atome d'hydrogène, un radical alcoyle linéaire, ramifié ou cyclique renfermant jusqu'à 8 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou naphtyle ou un radical thiényle, furyle pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno [2,3-b] furanyle, 2H-furo [3,2-b]-pyranyle, benzoxazolyle ou morpholinyle, le radical R étant éventuellement substitué par un ou plusieurs substituants choisis dans le groupe constitué par le radical hydroxy, le radical acétoxy, le radical méthoxy ou le radical benzyloxy, les radicaux alcoyles linéaires, ramifiés ou cycliques, saturés ou insaturés, comportant jusqu'à 18 atomes de carbone, les atomes d'halogène, les groupements $CF_3$, $SCF_3$, $OCF_3$, $NO_2$, $NH_2$ ou C≡N, le radical phényle, les groupements acyles ou alcoxycarbonyles renfermant de 2 à 8 atomes de carbone et les groupements alcoylsulfonyles renfermant de 1 à 6 atomes de carbone caractérisé en ce que l'on soumet la 1,5-dihydro 5-hydroxy 2H-pyrrol 2-one de formule :

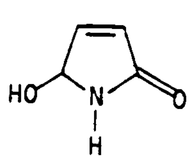

à l'action d'un alcool de formule (II) :

R'-OH (II)

R' étant défini comme précédemment, pour obtenir le composé de formule (III) :

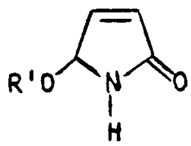

(III)

que l'on soumet à l'action d'un composé de formule (IV) :

$RSO_2$-Hal (IV)

dans laquelle Hal représente un atome de chlore ou de brome et R conserve sa signification précédente, pour obtenir le composé de formule (I) correspondant.

**2.** Procédé selon la revendication 1, caractérisé en ce que l'on utilise au départ un composé de formule (IV) dans laquelle R représente un radical phényle éventuellement substitué.

**3.** Procédé selon la revendication 1 ou 2, caractérisé en ce que l'on utilise au départ un composé de formule (II) dans laquelle R' représente un radical alcoyle linéaire, ramifié ou cyclique, renfermant jusqu'à 8 atomes de carbone.

**4.** Procédé selon la revendication 3, caractérisé en ce que l'on utilise au départ un composé de formule (III) dans laquelle R' représente un radical n-butyle ou n-pentyle.

**5.** Procédé selon la revendication 1, pour la préparation des composés dont les noms suivent :
- la 1-benzènesulfonyl 1,5-dihydro 5-n-butoxy 2H-pyrrol 2-one,
- la 1-benzènesulfonyl 1,5-dihydro 5-n-pentyloxy 2H-pyrrol 2-one,
- la 1-(3-trifluorométhyl benzènesulfonyl) 1,5-dihydro 5-n-butoxy 2H-pyrrol 2-one,

caractérisé en ce que l'on utilise au départ un composé de formule (IV) dans laquelle R représente respectivement un radical phényle ou 3-trifluorométhyl phényle et un composé de formule (II) dans laquelle R' représente respectivement un radical n-butyle ou n-pentyle.

**6.** Procédé de préparation de compositions pharmaceutiques, caractérisé en ce que l'on met, à titre de principe actif, l'un au moins des produits de formule (I), tels que définis à la revendication 1, sous une forme destinée à cet usage.

**7.** Procédé de préparation de compositions pharmaceutiques, caractérisé en ce que l'on met, à titre de principe actif, l'un au moins des produits de formule (I), tels que définis à l'une quelconque des revendications 2 à 4, sous une forme destinée à cet usage.

**8.** Procédé de préparation de compositions pharmaceutiques, caractérisé en ce que l'on met, à titre de principe actif, l'un au moins des produits de formule (I), tels que définis à la revendication 5, sous une forme destinée à cet usage.

**Claims**
**Claims for the following Contracting States : AT, BE, CH, DE, FR, GB, IT, LI, LU, NL, SE**

**1.** The compounds corresponding to general formula (I):

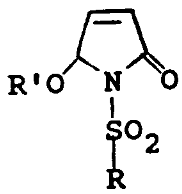

(I)

in which R' represents a hydrogen atom, a linear, branched or cyclic alkyl radical containing up to 8 carbon atoms, an alkenyl radical containing 2 to 8 carbon atoms, an acyl radical containing 1 to 6 carbon atoms or a benzyl or phenethyl radical and R represents a phenyl or naphthyl radical or one of the following radicals: thienyl, furyl, pyranyl, pyridyl, benzofuranyl, isobenzofuranyl, chromanyl, isochromanyl, chromenyl, xanthenyl, phenoxathienyl, oxazolyl, isoxazolyl, furazanyl, phenoxazinyl, thieno-[2,3-b]-furanyl, 2H-furo-[3,2-b]-pyranyl, benzoxazolyl or morpholinyl, the R radical being optionally substituted by one or more substituents chosen from the group constituted by the hydroxy radical, the acetoxy radical, the methoxy radical or the benzyloxy radical, saturated or unsaturated linear, branched or cyclic alkyl radicals containing up to 18 carbon atoms, halogen atoms, $CF_3$, $SCF_3$, $OCF_3$, $NO_2$, $NH_2$ or C≡N groups, the phenyl radical, acyl or alkoxycarbonyl groups containing 2 to 8 carbon atoms and alkylsulphonyl groups containing 1 to 6 carbon atoms.

**2.** The compounds of formula (I) as defined in claim 1, in which R represents a phenyl radical optionally substituted by one or more substituents defined in claim 1.

**3.** The compounds of formula (I) as defined in claim 1, in which R' represents a linear, branched or cyclic alkyl radical containing up to 8 carbon atoms.

**4.** The compounds of formula (I) as defined in claim 3, in which R' represents an n-butyl or n-pentyl radical.

**5.** The compounds of formula (I) as defined in claim 4, of which the names follow:
- 1-(benzenesulphonyl)-1,5-dihydro-5-n-butoxy-2H-pyrrol-2-one,
- 1-(benzenesulphonyl)-1,5-dihydro-5-n-pentyloxy-2H-pyrrol-2-one,
- 1-(3-trifluoromethyl benzenesulphonyl)-1,5-dihydro-5-n-butoxy-2H-pyrrol-2-one.

**6.** As medicaments, the compounds of formula (I) as defined in any one of claims 1 to 5.

**7.** As medicaments, the compounds defined in claim 5.

**8.** The pharmaceutical compositions containing as active ingredient at least one medicament defined in claim 6 or 7.

**9.** Preparation process for compounds of formula (I) as defined in any one of claims 1 to 5, characterized in that 1,5-dihydro-5-hydroxy-2H-pyrrol-2-one of formula:

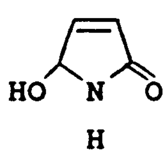

is subjected to the action of an alcohol of formula (II):

R'-OH (II)

R' being defined as previously, in order to obtain the compound of formula (III):

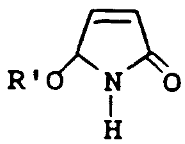

(III)

which is subjected to the action of a compound of formula (IV):

$RSO_2$-Hal (IV)

in which Hal represents a chlorine or bromine atom and R keeps its previous meaning, in order to obtain the corresponding compound of formula (I).

**Claims for the following Contracting State : ES**

**1.** Preparation process for compounds corresponding to general formula (I):

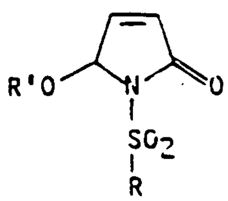

(I)

in which R' represents a hydrogen atom, a linear, branched or cyclic alkyl radical containing up to 8 carbon atoms, an alkenyl radical containing 2 to 8 carbon atoms, an acyl radical containing 1 to 6 carbon atoms or a benzyl or phenethyl radical and R represents a phenyl or naphthyl radical or one of the following radicals: thienyl, furyl, pyranyl, pyridyl, benzofuranyl, isobenzofuranyl, chromanyl, isochromanyl, chromenyl, xanthenyl, phenoxathienyl, oxazolyl, isoxazolyl, furazanyl, phenoxazinyl, thieno-[2,3-b]-furanyl, 2H-furo-[3,2-b]-pyranyl, benzoxazolyl or morpholinyl, the R radical being optionally substituted by one or more substituents chosen from the group constituted by the hydroxy radical, the acetoxy radical, the methoxy radical or the benzyloxy radical, saturated or unsaturated linear, branched or cyclic alkyl radicals containing up to 18 carbon atoms, halogen atoms, $CF_3$, $SCF_3$, $OCF_3$, $NO_2$, $NH_2$ or C≡N groups, the phenyl radical, acyl or alkoxycarbonyl groups containing 2 to 8 carbon atoms and alkylsulphonyl groups containing 1 to 6 carbon atoms, characterized in that 1,5-dihydro-5-hydroxy-2H-pyrrol-2-one of formula:

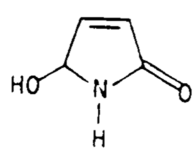

is subjected to the action of an alcohol of formula (II):

R'-OH (II)

R' being defined as previously, in order to obtain the compound of formula (III):

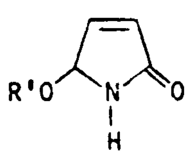

(III)

which is subjected to the action of a compound of formula (IV):

$RSO_2$-Hal (IV)

in which Hal represents a chlorine or bromine atom and R keeps its previous meaning, in order to obtain the corresponding compound of formula (I).

**2.** Process according to claim 1, characterized in that at the start a compound of formula (IV) is used in which R represents an optionally substituted phenyl radical.

**3.** Process according to claim 1 or 2, characterized in that at the start a compound of formula (II) is used in which R' represents a linear, branched or cyclic alkyl radical containing up to 8 carbon atoms.

**4.** Process according to claim 3, characterized in that at the start a compound of formula (III) is used in which R' represents an n-butyl or n-pentyl radical.

**5.** Process according to claim 1, for the preparation of compounds of which the names follow:
- 1-benzenesulphonyl-1,5-dihydro-5-n-butoxy-2H-pyrrol-2-one,
- 1-benzenesulphonyl-1,5-dihydro-5-n-pentyloxy-2H-pyrrol-2-one,
- 1-(3-trifluoromethyl benzenesulphonyl)-1,5-dihydro-5-n-butoxy-2H-pyrrol-2-one,

characterized in that at the start a compound of formula (IV) is used in which R represents respectively a phenyl or 3-trifluoromethyl phenyl radical and a compound of formula (II) in which R' represents respectively an n-butyl or n-pentyl radical.

**Claims for the following Contracting State : GR**

**1.** Preparation process for compounds corresponding to general formula (I):

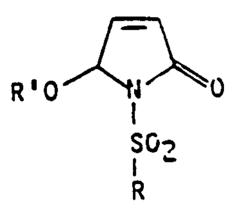

(I)

in which R' represents a hydrogen atom, a linear, branched or cyclic alkyl radical containing up to 8 carbon atoms, an alkenyl radical containing 2 to 8 carbon atoms, an acyl radical containing 1 to 6 carbon atoms or a benzyl or phenethyl radical and R represents a phenyl or naphthyl radical or one of the following radicals: thienyl, furyl, pyranyl, pyridyl, benzofuranyl, isobenzofuranyl, chromanyl, isochromanyl, chromenyl, xanthenyl, phenoxathienyl, oxazolyl, isoxazolyl, furazanyl, phenoxazinyl, thieno-[2,3-b]-furanyl, 2H-furo-[3,2-b]-pyranyl, benzoxazolyl or morpholinyl, the R radical being optionally substituted by one or more substituents chosen from the group constituted by the hydroxy radical, the acetoxy radical, the methoxy radical or the benzyloxy radical, saturated or unsaturated linear, branched or cyclic alkyl radicals containing up to 18 carbon atoms, halogen atoms, $CF_3$, $SCF_3$, $OCF_3$, $NO_2$, $NH_2$ or C≡N groups, the phenyl radical, acyl or alkoxycarbonyl groups containing 2 to 8 carbon atoms and alkylsulphonyl groups containing 1 to 6 carbon atoms, characterized in that 1,5-dihydro-5-hydroxy-2H-pyrrol-2-one of formula:

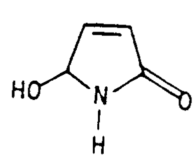

is subjected to the action of an alcohol of formula (II):

R'-OH (II)

R' being defined as previously, in order to obtain the compound of formula (III):

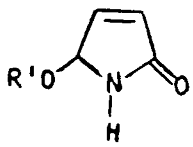

(III)

which is subjected to the action of a compound of formula (IV):

$RSO_2$-Hal (IV)

in which Hal represents a chlorine or bromine atom and R keeps its previous meaning, in order to obtain the corresponding compound of formula (I).

**2.** Process according to claim 1, characterized in that at the start a compound of formula (IV) is used in which R represents an optionally substituted phenyl radical.

**3.** Process according to claim 1 or 2, characterized in that at the start a compound of formula (II) is used in which R' represents a linear, branched or cyclic alkyl radical containing up to 8 carbon atoms.

**4.** Process according to claim 3, characterized in that at the start a compound of formula (III) is used in which R' represents an n-butyl or n-pentyl radical.

**5.** Process according to claim 1, for the preparation of compounds of which the names follow:
- 1-benzenesulphonyl-1,5-dihydro-5-n-butoxy-2H-pyrrol-2-one,
- 1-benzenesulphonyl-1,5-dihydro-5-n-pentyloxy-2H-pyrrol-2-one,
- 1-(3-trifluoromethyl benzenesulphonyl)-1,5-dihydro-5-n-butoxy-2H-pyrrol-2-one,

characterized in that at the start a compound of formula (IV) is used in which R represents respectively a phenyl or 3-trifluoromethyl phenyl radical and a compound of formula (II) in which R' represents respectively an n-butyl or n-pentyl radical.

**6.** Preparation process for pharmaceutical compositions, characterized in that at least one of the products of formula (I), as defined in claim 1, is used as active ingredient in a form intended for this use.

**7.** Preparation process for pharmaceutical compositions, characterized in that at least one of the products of formula (I), as defined in any one of claims 2 to 4, is used as active ingredient in a form intended for this use.

**8.** Preparation process for pharmaceutical compositions, characterized in that at least one of the products of formula (I), as defined in claim 5, is used as active ingredient in a form intended for this use.

**Patentansprüche**

**Patentansprüche für folgende Vertragsstaaten : AT, BE, CH, DE, FR, GB, IT, LI, LU, NL, SE**

**1.** Verbindungen der allgemeinen Formel (I)

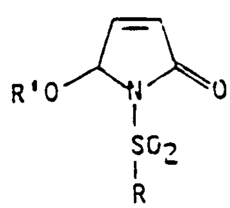

(I)

worin R' für ein Wasserstoffatom, einen linearen, verzweigten oder cyclischen Alkylrest mit bis zu 8 Kohlenstoffatomen, einen Alkenylrest mit 2 bis 8 Kohlenstoffatomen, einen Acylrest mit 1 bis 6 Kohlenstoffatomen oder einen Benzyl- oder Phenethylrest steht und R einen Phenyl- oder Naphthylrest oder einen Thienyl-, Furyl-, Pyranyl-, Pyridyl-, Benzofuranyl-, Isobenzofuranyl-, Chromanyl-, Isochromanyl-, Chromenyl-, Xanthenyl-, Phenoxathienyl-, Oxazolyl-, Isoxazolyl-, Furazanyl-,

Phenoxazinyl-, Thieno[2,3-b]furanyl-, 2H-Furo[3,2-b]pyranyl-, Benzoxazolyl- oder Morpholinylrest bedeutet, wobei der Rest R gegebenenfalls durch einen oder mehrere Substituenten substituiert ist, ausgewählt unter der Hydroxygruppe, dem Acetoxyrest, dem Methoxyrest oder dem Benzyloxyrest, den gesättigten oder ungesättigten, linearen, verzweigten oder cyclischen Alkylresten mit bis zu 18 Kohlenstoffatomen, den Halogenatomen, den Gruppen $CF_3$, $SCF_3$, $OCF_3$, $NO_2$, $NH_2$ oder C≡N, der Phenylgruppe, den Acyloder Alkoxycarbonylresten mit 2 bis 8 Kohlenstoffatomen und den Alkylsulfonylgruppen mit 1 bis 6 Kohlenstoffatomen.

2. Verbindungen der Formel (I), wie in Anspruch 1 definiert, worin R für einen Phenylrest steht, der gegebenenfalls durch einen oder mehrere Substituenten, wie in Anspruch 1 definiert, substituiert ist.

3. Verbindungen der Formel (I), wie in Anspruch 1 definiert, worin R' für einen linearen, verzweigten oder cyclischen Alkylrest mit bis zu 8 Kohlenstoffatomen steht.

4. Verbindungen der Formel (I) gemäß Anspruch 3, worin $R^1$ für einen n-Butyl- oder n-Pentylrest steht.

5. Verbindungen der Formel (I), wie in Anspruch 4 definiert, mit den folgenden Bezeichnungen:
1-(Benzolsulfonyl)-1,5-dihydro-5-n-butoxy-2H-pyrrol-2-on,
1-(Benzolsulfonyl)-1,5-dihydro-5-n-pentyloxy-2H-pyrrol-2-on,
1-(3-Trifluormethyl)-benzolsulfonyl-1,5-dihydro-5-n-butoxy-2H-pyrrol-2-on.

6. Als Arzneimittel die Verbindungen der Formel (I), wie in einem der Ansprüche 1 bis 5 definiert.

7. Als Arzneimittel die Verbindungen, wie in Anspruch 5 definiert.

8. Pharmazeutische Zusammensetzungen, enthaltend als Wirkstoff zumindest ein Arzneimittel, wie in Anspruch 6 oder 7 definiert.

9. Verfahren zur Herstellung der Verbindungen der Formel (I), wie in einem der Ansprüche 1 bis 5 definiert, dadurch gekennzeichnet, daß man das 1,5-Dihydro-5-hydroxy-2H-pyrrol-2-on der Formel

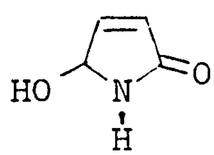

der Einwirkung eines Alkohols der Formel (II)

R'-OH (II)

worin R' wie vorstehend definiert ist, unterzieht, um zu der Verbindung der Formel (III)

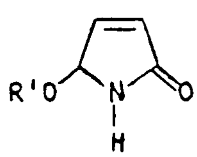

(III)

zu gelangen, die man der Einwirkung einer Verbindung der Formel (IV)

$RSO_2$-Hal (IV)

unterzieht, worin Hal für ein Chlor- oder Bromatom steht und R die vorstehend angegebene Bedeutung besitzt, um die entsprechende Verbindung der Formel (I) zu erhalten.

**Patentansprüche für folgenden Vertragsstaat : ES**

1. Verfahren zur Herstellung der Verbindungen der allgemeinen Formel (I)

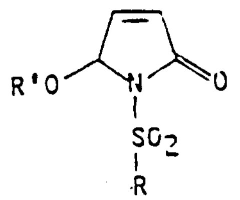

(I)

worin R' für ein Wasserstoffatom, einen linearen, verzweigten oder cyclischen Alkylrest mit bis zu 8 Kohlenstoffatomen, einen Alkenylrest mit 2 bis 8 Kohlenstoffatomen, einen Acylrest mit 1 bis 6 Kohlenstoffatomen oder einen Benzyl- oder Phenethylrest steht und R einen Phenyl- oder Naphthylrest oder einen Thienyl-, Furyl-, Pyranyl-, Pyridyl-, Benzofuranyl-, Isobenzofuranyl-, Chromanyl-, Isochromanyl-, Chromenyl-, Xanthenyl-, Phenoxathienyl-, Oxazolyl-, Isoxazolyl-, Furazanyl-, Phenoxazinyl-, Thieno[2,3-b]furanyl-, 2H-Furo[3,2-b]pyranyl-, Benzoxazolyl- oder Morpholinylrest bedeutet, wobei der Rest R gegebenenfalls durch einen oder mehrere Substituenten substituiert ist, ausgewählt unter der Hydroxygruppe, dem Acetoxyrest, dem Methoxyrest oder dem Benzyloxyrest, den gesättigten oder ungesättigten, linearen, verzweigten oder cyclischen Alkylresten mit bis zu 18 Kohlenstoffatomen, den Halogenatomen, den Gruppen $CF_3$, $SCF_3$, $OCF_3$, $NO_2$, $NH_2$ oder C≡N, der Phenylgruppe, den Acyloder Alkoxycarbonylresten mit 2 bis 8 Kohlenstoffatomen und den Alkylsulfonylgruppen mit 1 bis 6 Kohlenstoffatomen,
dadurch **gekennzeichnet,** daß man das 1,5-Dihydro-5-hydroxy-2H-pyrrol-2-on der Formel

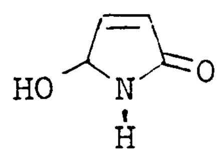

der Einwirkung eines Alkohols der Formel (II)

R'-OH (II)

worin R' wie vorstehend definiert ist, unterzieht, um zu der Verbindung der Formel (III)

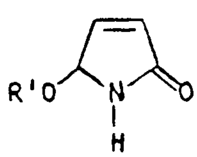

(III)

zu gelangen, die man der Einwirkung einer Verbindung der Formel (IV)

$RSO_2$-Hal (IV)

unterzieht, worin Hal für ein Chlor- oder Bromatom steht und R die vorstehend angegebene Bedeutung besitzt, um die entsprechende Verbindung der Formel (I) zu erhalten.

2. Verfahren gemäß Anspruch 1, dadurch gekennzeichnet, daß man von einer Verbindung der Formel (IV) ausgeht, worin R für einen gegebenenfalls substituierten Phenylrest steht.

3. Verfahren gemäß Anspruch 1 oder 2, dadurch gekennzeichnet, daß man von einer Verbindung der Formel (II) ausgeht, worin R' für einen linearen, verzweigten oder cyclischen Alkylrest mit bis zu 8 Kohlenstoffatomen steht.

4. Verfahren gemäß Anspruch 3, dadurch gekennzeichnet, daß man von einer Verbindung der Formel (III) ausgeht, worin R' für einen n-Butyl- oder n-Pentylrest steht.

5. Verfahren gemäß Anspruch 1 zur Herstellung der Verbindungen mit den folgenden Bezeichnungen:
1-Benzolsulfonyl-1,5-dihydro-5-n-butoxy-2H-pyrrol-2-on,
1-Benzolsulfonyl-1,5-dihydro-5-n-pentyloxy-2H-pyrrol-2-on,
1-(3-Trifluormethylbenzolsulfonyl)-1,5-dihydro5-n-butoxy-2H-pyrrol-2-on,
dadurch gekennzeichnet, daß man von einer Verbindung der Formel (IV), worin R für einen Phenyl- bzw. 3-Trifluormethylphenylrest steht, und einer Verbindung der Formel (II), worin R' für einen n-Butyl- bzw. n-Pentylrest steht, ausgeht.

**Patentansprüche für folgenden Vertragsstaat : GR**

1. Verfahren zur Herstellung der Verbindungen der allgemeinen Formel (I)

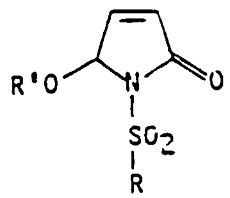

(I)

worin R' für ein Wasserstoffatom, einen linearen, verzweigten oder cyclischen Alkylrest mit bis zu 8 Kohlenstoffatomen, einen Alkenylrest mit 2 bis 8 Kohlenstoffatomen, einen Acylrest mit 1 bis 6 Kohlenstoffatomen oder einen Benzyl- oder Phenethylrest steht und R einen Phenyl- oder Naphthylrest oder einen Thienyl-, Furyl-, Pyranyl-, Pyridyl-, Benzofuranyl-, Isobenzofuranyl-, Chromanyl-, Isochromanyl-, Chromenyl-, Xanthenyl-, Phenoxathienyl-, Oxazolyl-, Isoxazolyl-, Furazanyl-, Phenoxazinyl-, Thieno[2,3-b]furanyl-, 2H-Furo[3,2-b]pyranyl-, Benzoxazolyl- oder Morpholinylrest bedeutet, wobei der Rest R gegebenenfalls durch einen oder mehrere Substituenten substituiert ist, ausgewählt unter der Hydroxygruppe, dem Acetoxyrest, dem Methoxyrest oder dem Benzyloxyrest, den gesättigten oder ungesättigten, linearen, verzweigten oder cyclischen Alkylresten mit bis zu 18 Kohlenstoffatomen, den Halogenatomen, den Gruppen $CF_3$, $SCF_3$, $OCF_3$, $NO_2$, $NH_2$ oder C≡N, der Phenylgruppe, den Acyloder Alkoxycarbonylresten mit 2 bis 8 Kohlenstoffatomen und den Alkylsulfonylgruppen mit 1 bis 6 Kohlenstoffatomen,
dadurch **gekennzeichnet,** daß man das 1,5-Dihydro-5-hydroxy-2H-pyrrol-2-on der Formel

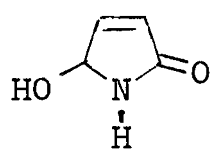

der Einwirkung eines Alkohols der Formel (II)

R'-OH (II)

worin R' wie vorstehend definiert ist, unterzieht, um zu der Verbindung der Formel (III)

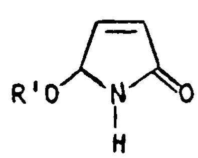

(III)

zu gelangen, die man der Einwirkung einer Verbindung der Formel (IV)

$RSO_2$-Hal (IV)

unterzieht, worin Hal für ein Chlor- oder Bromatom steht und R die vorstehend angegebene Bedeutung besitzt, um die entsprechende Verbindung der Formel (I) zu erhalten.

2. Verfahren gemäß Anspruch 1, dadurch gekennzeichnet, daß man von einer Verbindung der Formel (IV) ausgeht, worin R für einen gegebenenfalls substituierten Phenylrest steht.

3. Verfahren gemäß Anspruch 1 oder 2, dadurch gekennzeichnet, daß man von einer Verbindung der Formel (II) ausgeht, worin R' für einen linearen, verzweigten oder cyclischen Alkylrest mit bis zu 8 Kohlenstoffatomen steht.

4. Verfahren gemäß Anspruch 3, dadurch gekennzeichnet, daß man von einer Verbindung der Formel (III) ausgeht, worin R' für einen n-Butyl- oder n-Pentylrest steht.

5. Verfahren gemäß Anspruch 1 zur Herstellung der Verbindungen mit den folgenden Bezeichnungen:
   1-Benzolsulfonyl-1,5-dihydro-5-n-butoxy-2H-pyrrol-2-on,
   1-Benzolsulfonyl-1,5-dihydro-5-n-pentyloxy-2H-pyrrol-2-on,
   1-(3-Trifluormethylbenzolsulfonyl)-1,5-dihydro-5-n-butoxy-2H-pyrrol-2-on,
   dadurch gekennzeichnet, daß man von einer Verbindung der Formel (IV), worin R für einen Phenyl- bzw. 3-Trifluormethylphenylrest steht, und einer Verbindung der Formel (II), worin R' für einen n-Butyl- bzw. n-Pentylrest steht, ausgeht.

6. Verfahren zur Herstellung von pharmazeutischen Zusammensetzungen, dadurch gekennzeichnet, daß man als Wirkstoff zumindest eines der Produkte der Formel (I), wie in Anspruch 1 definiert, in eine für diese Verwendung bestimmte Form überführt.

7. Verfahren zur Herstellung von pharmazeutischen Zusammensetzungen, dadurch gekennzeichnet, daß man als Wirkstoff zumindest eines der Produkte der Formel (I), wie in einem der Ansprüche 2 bis 4 definiert, in eine für diese Verwendung bestimmte Form überführt.

8. Verfahren zur Herstellung von pharmazeutischen Zusammensetzungen, dadurch gekennzeichnet, daß man als Wirkstoff zumindest eines der Produkte der Formel (I), wie in Anspruch 5 definiert, in eine für diese Verwendung bestimmte Form überführt.